# EUROPÄISCHE PATENTSCHRIFT

(11) **EP 0 672 746 B1**
(45) Veröffentlichungstag und Bekanntmachung des Hinweises auf die Patenterteilung: **29.05.2002**
(21) Anmeldenummer: 95103365.3
(22) Anmeldetag: 09.03.1995
(51) Int. Cl.: C11B 9/00, C07C 251/36, C07C 251/42, C07C 251/44, C07C 251/48

(54) **Oximether und diese enthaltende Riech- und Geschmackstoffkompositionen**
Oxime ethers and perfume and flavouring compositions containing them
Ethers d'oximes et compositions parfumantes et aromatisantes les contenant

(30) Priorität: 18.03.1994 CH 81394
(43) Veröffentlichungstag der Anmeldung: 20.09.1995
(73) Patentinhaber: Givaudan SA, 1214 Vernier-Genève (CH)
(72) Erfinder: Kaiser, Roman, CH-8610 Uster (CH); Naegeli, Peter, CH-5430 Wettingen (CH); Nussbaumer, Cornelius, CH-8603 Schwerzenbach (CH)
(74) Vertreter: Patentanwälte Schaad, Balass, Menzl & Partner AG

(56) Entgegenhaltungen:
- EP-A- 0 071 248
- EP-A- 0 085 352
- EP-A- 0 214 588
- DE-A- 1 692 002
- US-A- 2 791 611
- JOURNAL OF THE CHEMICAL SOCIETY, Bd.71, 1897 Seiten 1030 - 1050 M. O. FORSTER 'Camhporoxime. Part II. The ethers of camphoroxime'
- ORGANIC MAGNETIC RESONANCE, Bd.21, Nr.6, 1983 Seiten 357 - 360 M. ROUILLARD ET AL. '1H NMR utilization of through-space effects'
- JOURNAL OF ORGANIC CHEMISTRY, Bd.36, Nr.22, 1971, EASTON US Seiten 3467 - 69 SOU-YIE CGU; D. A. COVIELLO 'Preparation of 2-alkoxyiminoalkyl bromides by the bromination of O-alkyl oximes with N-bromsuccinimide'
- TETRAHEDRON LETTERS, Bd.45, 1975, OXFORD GB Seiten 3889 - 3892 TH. A. SPENCER, CH. W. LEONG 'Regioselectivity in .alpha. proton abstraction from ketone methoxims'
- JOURNAL OF THE CHEMICAL SOCIETY, CHEMICAL COMMUNICATIONS, 1976, LETCHWORTH GB Seiten 674 - 675 R. R. FRASER, K. I. DHAWAN 'Exclusive syn-axial alkylation of O-methyl oximes resulting from an orbital symmetry effect'
- JOURNAL OF ORGANIC CHEMISTRY, Bd.38, Nr.1, 1973, EASTON US Seiten 56 - 59 E. G. BOZZI ET AL. 'Reactions of .alpha.,.beta.-dibromo oximes and related compounds with nitrosylchloride'
- JOURNAL OF ORGANIC CHEMISTRY, Bd.36, Nr.14, 1971, EASTON US Seiten 1931 - 1933 S. G. SMITH, M. P. HANSON 'Control of site of alkylation of ambident anions'
- CHEMISTRY LETTERS, 1980, TOKYO JP Seiten 869 - 870 H. SHINOZAKI ET AL. 'The preparation of oxime ethers under phase transfer condition'
- TETRAHEDRON LETTERS, Bd.27, 1986, OXFORD GB Seiten 2199 - 2202 E. J. COREY ET AL. 'A new synthetic route to prostaglandins'
- JOURNAL OF THE CHEMICAL SOCIETY, PERKIN TRANSACTIONS 2, 1983, LETCHWORTH GB Seiten 721 - 725 J. LUB ET AL. 'Radical reactions of trimethylaluminium with sterically hindered chloro-nitroso-compound'
- JOURNAL OF ORGANIC CHEMISTRY, Bd.57, Nr.20, 1992, EASTON US Seiten 5528 - 5530 B. CAPON ET AL. 'Tautomerization of 1-(N-methoxyamino)cycloheptadien-1-one O-methyloxime: A regiospecific enamine-imine conversion'
- CHEMICAL AND PHARMACEUTICAL BULLETIN., Bd.37, Nr.6, 1989, TOKYO JP Seiten 1524 - 1533 K. SENO, S. HAGISHITA 'Thromboxane A2 receptor antagonist. III. Synthesis and pharmacological activity of 6,6-Dimethylbicyclo[3.1.1]heptane derivatives with a substituted sulfonylamino group at c-2'
- CHEMICAL AND PHARMACEUTICAL BULLETIN., Bd.36, Nr.8, 1988, TOKYO JP Seiten 3134 - 3137 H. IRIE ET AL. 'Synthesis of methoxyonychine and use of 1H- and 13C-nuclear magnetic resonance spectra for structure determination of geometrical isomers of indan-1-one oxime derivatives'
- JOURNAL OF ORGANIC CHEMISTRY, Bd.22, 1957, EASTON US Seiten 1024 - 1029 L. G. DONARUMA 'reduction of nitroparaffins by alkylation. I. Alkylation with trialkyl oxonium salts'

## Beschreibung

Die Erfindung betrifft Riech- und/oder Geschmackstoffkompositionen mit einem Gehalt an einer Verbindung der Formel 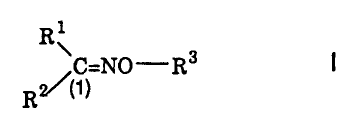
worin R¹, R² H, Alkyl, Alkenyl,
gegebenenfalls substituiertes Cycloalkyl,
gegebenenfalls substituiertes Cycloalkyl-alkyl,
gegebenenfalls substituiertes Cycloalkyl-alkenyl,
gegebenenfalls substituiertes Cycloalkenyl,
gegebenenfalls substituiertes Cycloalkenyl-alkyl oder
gegebenenfalls substituiertes Phenyl, substituiertes Benzyl,
substuiertes Phenethyl, Phenylalkenyl,
ein bicyclischer Rest,
oder R¹+R²+ Kohlenstoffatom C₁ zusammen gebenenfalls substituiertes Cycloalkyl oder ein bicyclischer Rest und
R³ C₁₋₄ Alkyl, C₂₋₄ Alkenyl oder Benzyl bedeuten.

a) Geeignete Reste R¹ und R² sind insbesondere gerade oder verzweigte Alkyl- bzw. Alkenylreste wie Methyl, Aethyl, Propyl, i-Propyl, Butyl, Pentyl, Hexyl, Allyl, Pentenyl, Hexenyl, Heptenyl und Octadienyl.
b) Beispiele für Cycloalkyl sind z.B. C₅-C₁₅-Reste, beispielsweise Cyclopentyl, Cyclohexyl, Cyclododecyl, Cyclopentadecyl, etc.
c) Beispiele für Cycloalkyl-alkyl sind C₅-C₆-Cycloalkyl-alkyl, beispielsweise Cyclopentyl-methyl, Cyclopentyl-ethyl, Cyclohexyl-methyl, Cyclohexyl-ethyl, etc.
d) Beispiele für Cycloalkyl-alkenyl sind C₅-C₇-Cycloalkylalkenyl, beispielsweise Cyclopentyl-äthenyl, Cyclohexyl-äthenyl, ∼n-propenyl, ∼n-butenyl, etc.
e) Beispiele für Cycloalkenyl sind Cyclopentenyl, Cyclohexenyl, Cycloheptenyl, etc.
f) Beispiele für Cycloalkenyl-alkyl sind C₅-C₇-Cycloalkenyl-alkyl, beispielsweise Cyclopentenyl-methyl, Cyclohexenyl-methyl, Cycloheptenylmethyl, etc.
g) Ein Beispiel eines geeigneten bicyclischen Restes ist Bicyclo-2,2,2-octenyl.
h) Beispiele für die Gruppierung R¹ + R² + C₁ sind Cyclohexyl, Cyclododecyl, Cyclopentadecyl, etc., Bicyclo-3,2,1-octyl, etc.
i) Beispiele für Phenylalkenyl sind Phenyl-alkenyl, z.B, Phenylaethenyl, Phenylpropenyl, Phenylbutenyl, etc.

Geeignete Substituenten für obige Reste sind insbesondere n-Alkyl- und n-Alkoxyreste, wie Methyl, Aethyl, Propyl, Isopropyl, etc., Methoxy, Ethoxy, Propoxy, i-Propoxy, etc., aber auch Hydroxy oder Thiomethyl, etc.

Spezifische Reste R³ sind Methyl, Aethyl, Propyl, Isopropyl, Butyl, etc., Allyl, Propenyl, i-Propenyl, 3-Methyl-butenyl, etc.

Die Oximether I sind herstellbar, in dem man entweder
a) die zugrunde liegende Oxoverbindung mit einem O-Alkyl-hydroxylamin kondensiert, oder
b) das entsprechende Oxim O-alkyliert.

Bei der Verfahrensvariante a), nämlich der Kondensation 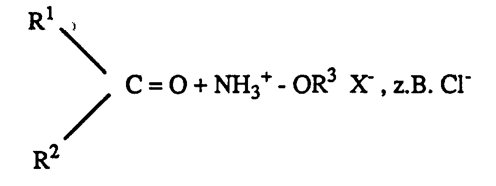 arbeitet man zweckmässigerweise in gepufferter wässriger Lösung, insbesondere bei einem pH- Wert von ca. 4,5 bis ca. 5,5, z.B. bewerkstelligt mittels eines Alkaliacetats oder mit Pyridin, und insbesondere bei Zimmertemperatur oder leicht erhöhter Temperatur.

Das Verhältnis der Reaktionspartner, nämlich der Carbonylverbindung und des Alkoxyaminhydrohalogenides in der wässrigen Lösung ist zweckmässigerweise ca. 1: ca 1-1,1.

Bei der Verfahrensvariante b), nämlich der Reaktion 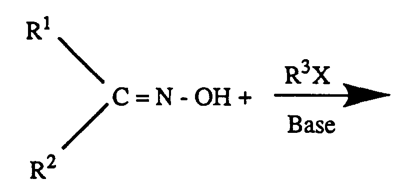 handelt es sich um eine übliche Alkylierung eines Oxims, zweckmässigerweise mittels eines Dialkylsulfats oder eines Alkylhalogenids, etc. bewerkstelligt.

Man arbeitet zweckmässigerweise in einem nicht - protischen organischen Lösungsmittel wie THF, DME, DMF, etc.

Als Base können beispielsweise Alkalihydroxide, wie KOH (vorzugsweise gepulvert), Alkalihydride, wie NaH, etc. eingesetzt werden.

Die erfindungsgemäss als Riech- und/oder Geschmackstoffe verwendeten Verbindungen der Formel I zeichnen sich durch angenehme, ausgesprochen natürlich wirkende grüne und fruchtige Noten aus; sie sind kräftig und frisch. Weitere (Neben)noten sind: würzig, holzig, Anis. Zudem zeichnen sie sich durch ein hohes Diffusionsvermögen, verbunden mit einer ungewöhnlichen Haftfestigkeit aus. Bereits in geringen Konzentrationen eingesetzt, verstärken und veredeln sie das Geruchsbild von Riechstoffkompositionen, insbesondere von Chypre- & von Fougère-noten, von fruchtigen, von blumigen, wie auch an holzige Töne erinnernden Parfums beziehungsweise Kompositionen oder Basen. Die Verbindungen I verbinden sich mit zahlreichen bekannten Riechstoffingredienzien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:
- Naturprodukte: Basilikumöl, Baummoos Absolue, Beifussöl, Bergamotteöl, Cassisknospen Absolue, Castoreum, Cedernholzöl, Ciste Labdanum, Civette, Corianderöl, Eichenmoos, Elemiöl, Fichtennadelöl, Galbanum, Geraniumöl, Gewürznelkenöl, Jasmin Absolue und sein synthetischer Ersatz, Jonquille Absolue, Kamillenöl, Labdanum, Lavendelöl, Mandarinenöl, Mastix Absolue, Mentha citrata-öl, Myrrhenöl, Palmarosaöl, Patchouliöl, Petitgrainöl Paraguay, Sandelholzöl, Thymianöl, Vassouraöl, Moschus Infusion, Styrax, Birkenteer, Vetiveröl, Weihrauch, Ylang-Ylang-Oel, Zitronenöl, Zibetöl, etc.
- Alkohole: Citronellöl, Dimetol® (2,6-Dimethyl-heptan-2-ol), Geraniol, cis-3-Hexanol, Linalool, Nonadyl® (6,8-Dimethyl-nonan-2-ol), Phenyläthylalkohol, Rhodinol, Sandela® (3-Isocamphyl-5-cyclohexanol), Sandalore® (3-Methyl-5 (2',2',3'-trimethyl-cyclopenta-3'-en-1'-yl)-pentan-2-ol), Terpineol, etc.
- Aldehyde: α-Amylzimtaldehyd, Cyclamenaldehyd, Decanal, Dodecanal, Heliotropin, α-Hexylzimtladehyd, Hydroxycitronellal, Lyral, Adoxal® (2,6,10-Trimethyl-9-en-1-al), Undecanal, ω-Undecylenaldehyd, Vanillin, etc.
- Ketone: Isoraldeine® (Isomethyl-α-jonon), α-Jonon, β-Jonon, 3-Phenylisocaranon, Vertofix® (=acetyliertes Cedernholzöl), p-Methylacetophenon, etc.
- Ester: Acetessigsäureäthylester, Amylsalicylat, Benzyl-acetat, Cedrylacetat, Cinnamylformiat, Citronellylacetat, Geranylacetat, cis-3-Hexenylacetat, cis-3-Hexanylbenzoat, Linalylacetat, Linalylanthranilat, Methyldihydrojasmonat, Methambrat® (1-Acetoxy- 1-methyl-2-sec.butylcyclohexan), Myraldylacetat® (4-(4-Methyl-3-pentenyl)-cyclohex-3-en-1-yl-carbinylacetat), Phenoxyäthylisobutyrat, Phenyläthyltiglat, Styrallylacetat, Terpenylacetat, 2,3,6,6-Tetramethylcyclohexy-2-encarbonsäure-äthylester, Vetivenylacetat, ortho-tert.-Butylcyclohexyl-acetat, etc.
- Verschiedene: Ambrettemoschus, Cumarin, Epoxycedren, Eugenol, Fixolide® (1,1,2,4,4,7-Hexamethyl-6-acetyl-1,2,3,4-tetrahydronaphthalin), Galaxolid® (1,3,4,6,7,8-Hexahydro-4,6,6,7,8,8-hexamethyl-cyclopenty-γ-2-benzopyran), Heliotropin, Indol, Indolen, Isoeugenol, Isobutylchinolin, Jasmonyl (1,3-Diacetoxy-nonan), Ketonmoschus, Limonen, p-Menthan-8-thiol-3-on, Madrox® (1-Methyl-cyclododecyl-methyläther), Methyleugenol, Musk 174® (12-Oxahexadecanolid), γ-Nonalacton, α-Undecalacton, etc.

Die Verbindungen der Formel I lassen sich in weiten Grenzen einsetzen, die beispielsweise von 0.1 (Detergentien) - 30 Gew.-% (alcoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0.1 und 25%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I können demgemäss bei der Herstellung von Kompositionen und - wie obige Zusammenstellung zeigt - unter Verwendung einer breiten Palette bekannter Riechstoffe bzw. Riechstoffgemische verwendet werden. Bei der Herstellung solcher Kompositionen können die oben angeführten bekannten Riechstoffe bzw. Riechstoffgemische nach (dem Parfümeur bekannter) Art und Weise verwendet werden, wie z.B. aus W.A. Poucher, Perfumes, Cosmetics, Soaps 2, 7. Auflage, Chapman und Hall, London 1974 hervorgehend.

Die Verbindungen der Formel I sind ebenfalls vorzüglich geeignet zur Verwendung in Aromen verschiedenster Art, insbesondere aber auch zur Aromatisierung von Tabak.

Als Geschmackstoffe können die Verbindungen I beispielsweise zur Erzeugung bzw. Verbesserung, Verstärkung, Steigerung oder Modifizierung von (tropischen) Fruchtaromen, z.B. Aprikosen-, Pfirsich-, Mango-, Guava-, Passionsfrucht-, Ananas-, Bananenaromen verwendet werden. Aber auch Nuss-, Kokosnuss-, Caramel-, Feldahorn-, Tee-, Tabakaromen kommen in Frage, Als Anwendungsgebiet dieser Aromen kommen beispielsweise Nahrungsmittel (Joghurt, Süsswaren etc.), Genussmittel (Tee, Tabak, etc.) und Getränke (Limonade etc.) in Frage.

Die ausgeprägten geschmacklichen Qualitäten der Verbindungen I (coumarinartig, laktonisch, nach Caramel; leicht fruchtig, frisch) ermöglichen die Verwendung als Aromastoffe in geringen Konzentrationen. Eine geeignete Dosierung umfasst beispielsweise den Bereich von 0.1 ppm - 100 ppm, vorzugsweise den Bereich von 0.5 ppm - 50 ppm im Fertigprodukt d.h. dem aromatisierten Nahrungsmittel, Genussmittel oder Getränk.

Bei der Aromatisierung von beispielsweise Mundpflegemitteln kann die Dosierung jedoch auch höher liegen und einen grösseren Bereich umfassen, beispielsweise den Bereich von 1 bis 1000 ppm, vorzugsweise 30-100 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0.1-10, insbesondere 0.5-3 Gew. %. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z.B. J. Merory, Food Flavorings, Composition, Manufacture and Use, Second Edition. The Avi Publishing Company, Inc., Westport, Conn. 1968, oder G. Fenaroli, Fenroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredienzien, etc. in Frage:
Gummi arabicum, Tragant, Salze oder Brauereihefe, Alginate, Carrageen oder ähnliche Absorbenzien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguenosin-5-phosphat (GMP); oder spezielle Aromastoffe, Wasser, Aethanol, Propylenglykol, Glycerin, etc.

Die im Rahmen dieser Erfindung als Riech- und/oder Geschmackstoffe verwendeten Oximether zeichnen sich insbesondere aus durch chemische Stabilität, insbesondere Oxydationsstabilität und Hydrolysestabilität, gute Applikationsfähigkeit, Farblosigkeit, nicht zu grosse Flüchtigkeit, geringe Toxizität.

In der folgenden Tabelle 1 sind eine Reihe der Oximäther I und ihre entsprechenden organoleptischen Eigenschaften aufgeführt.

Die gezeichneten Struktur formeln sollen die E- wie auch die Z-Form aller Verbindungen der Formel I umfassen.

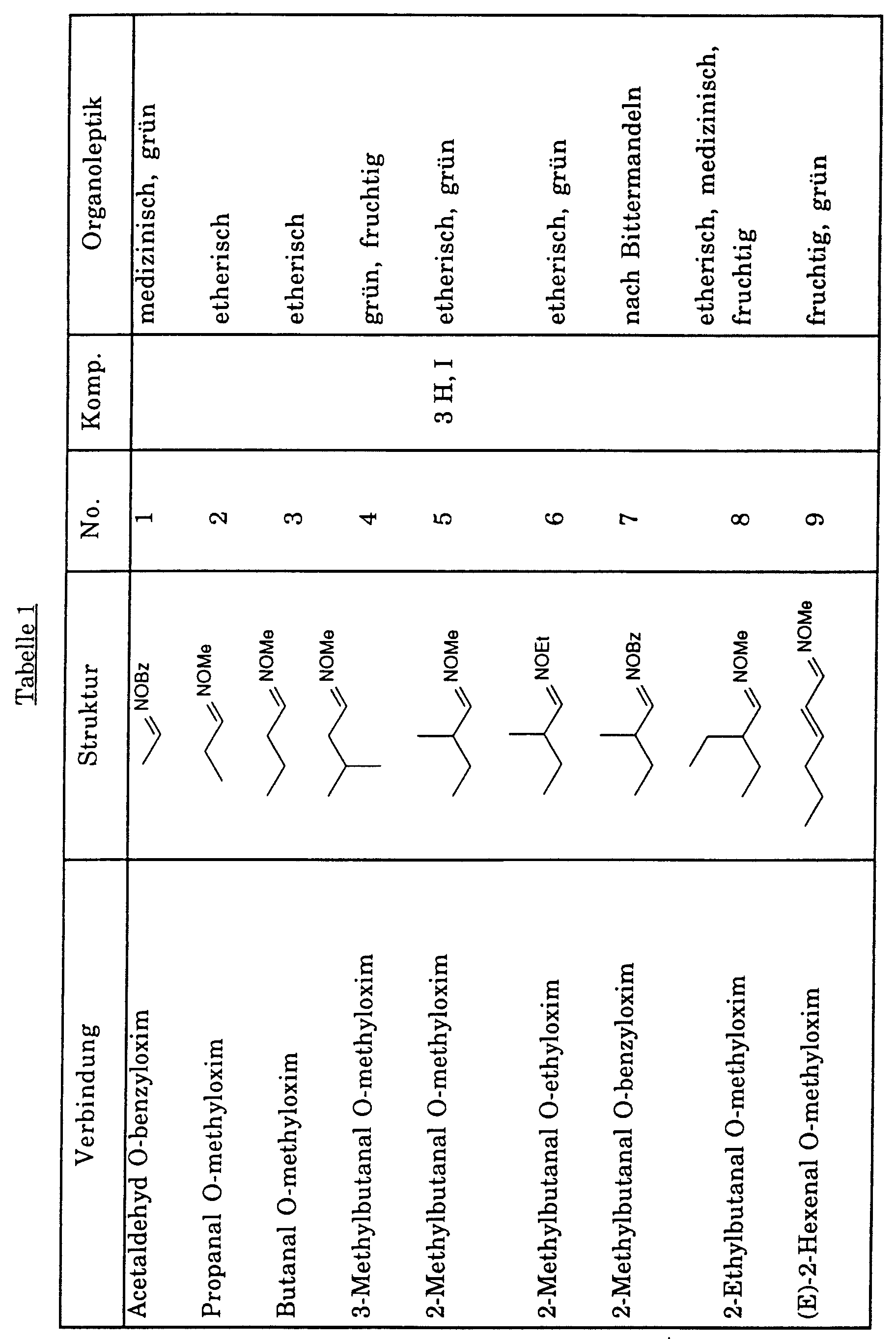

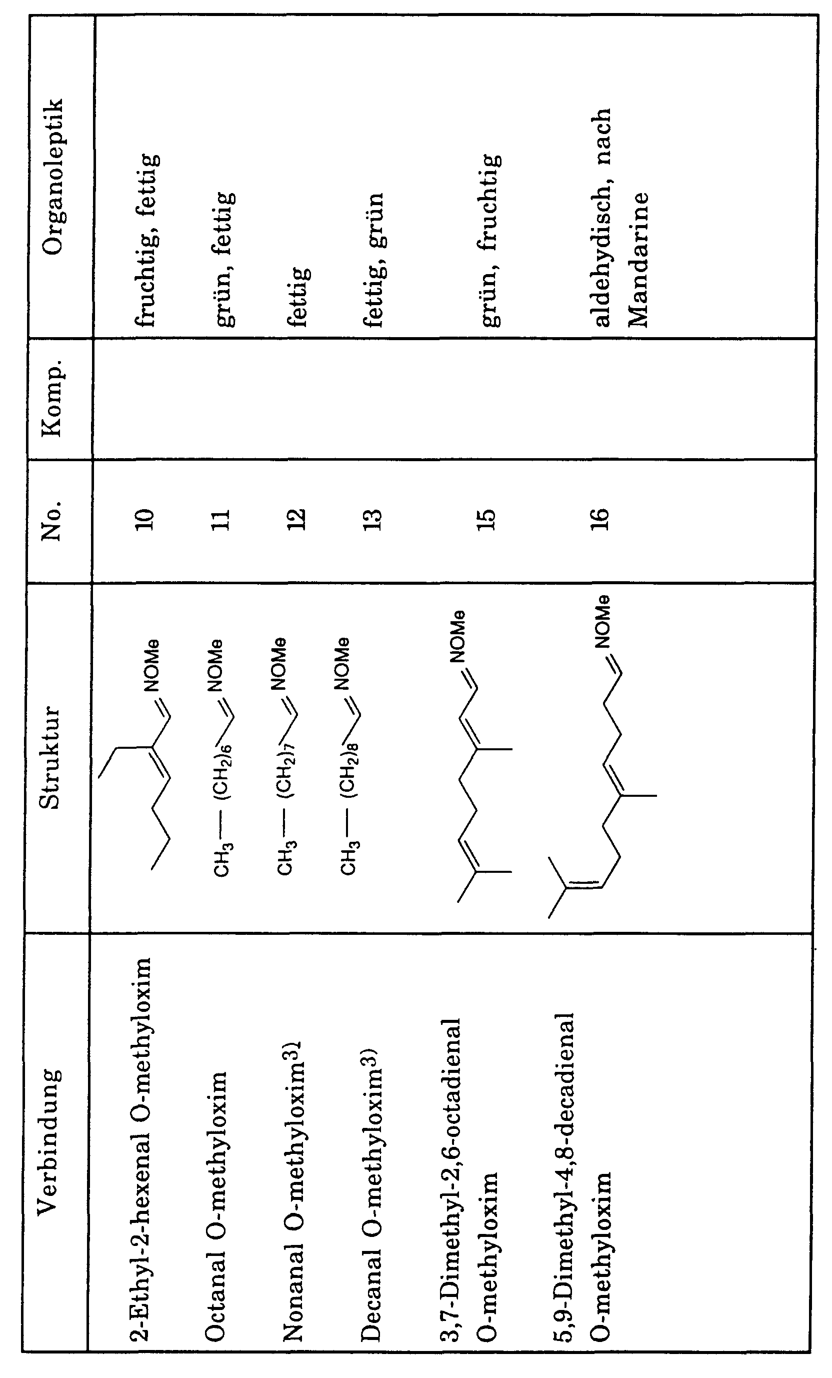

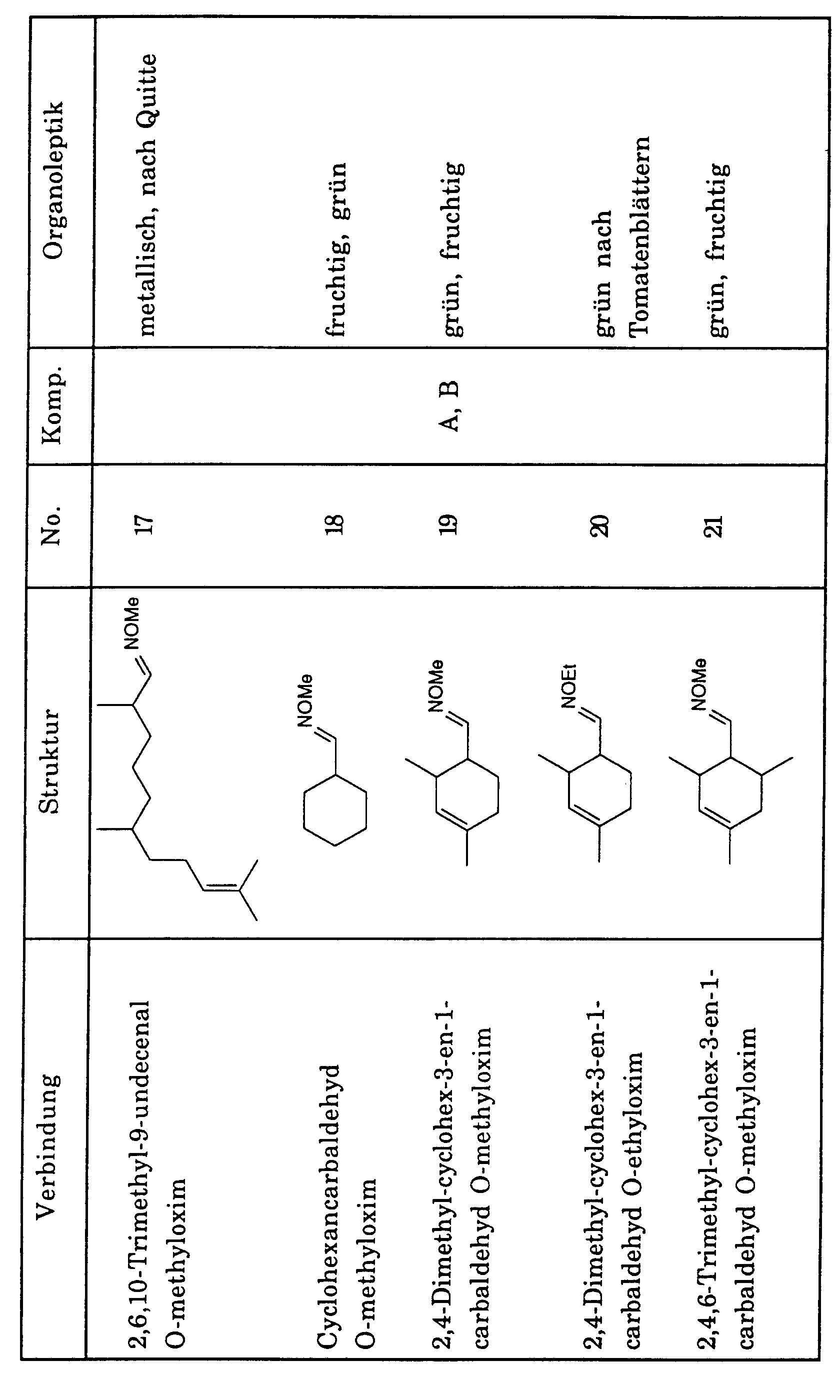

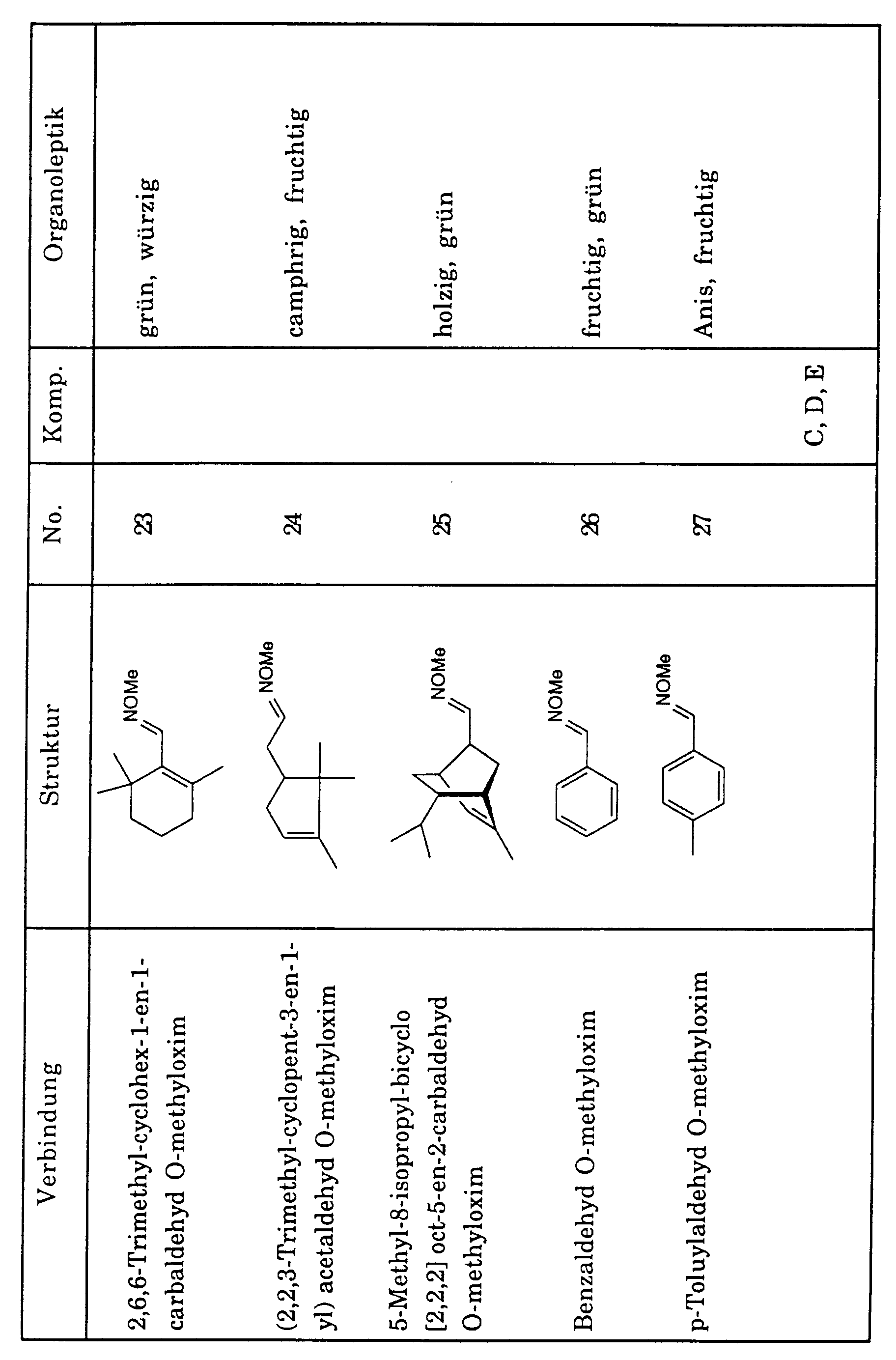

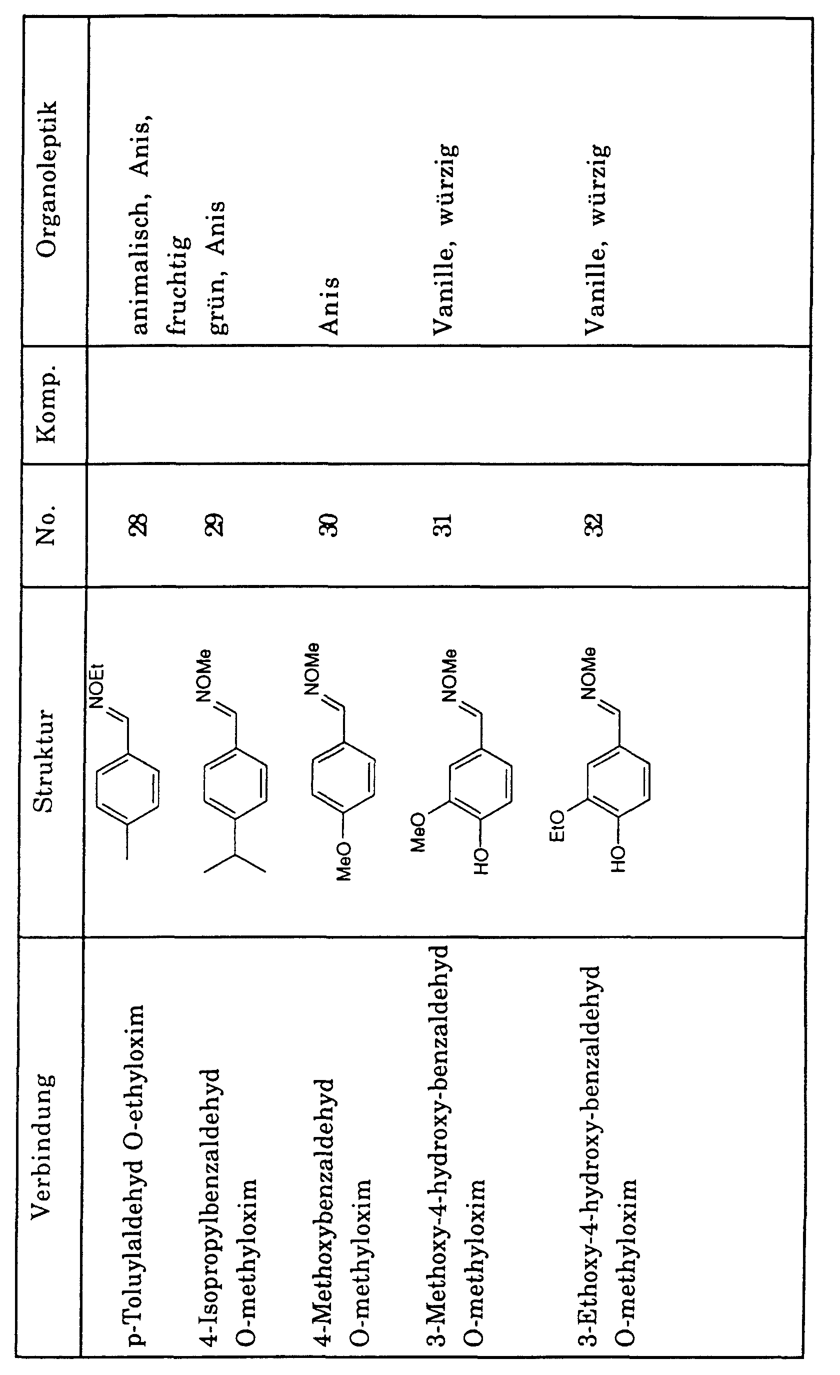

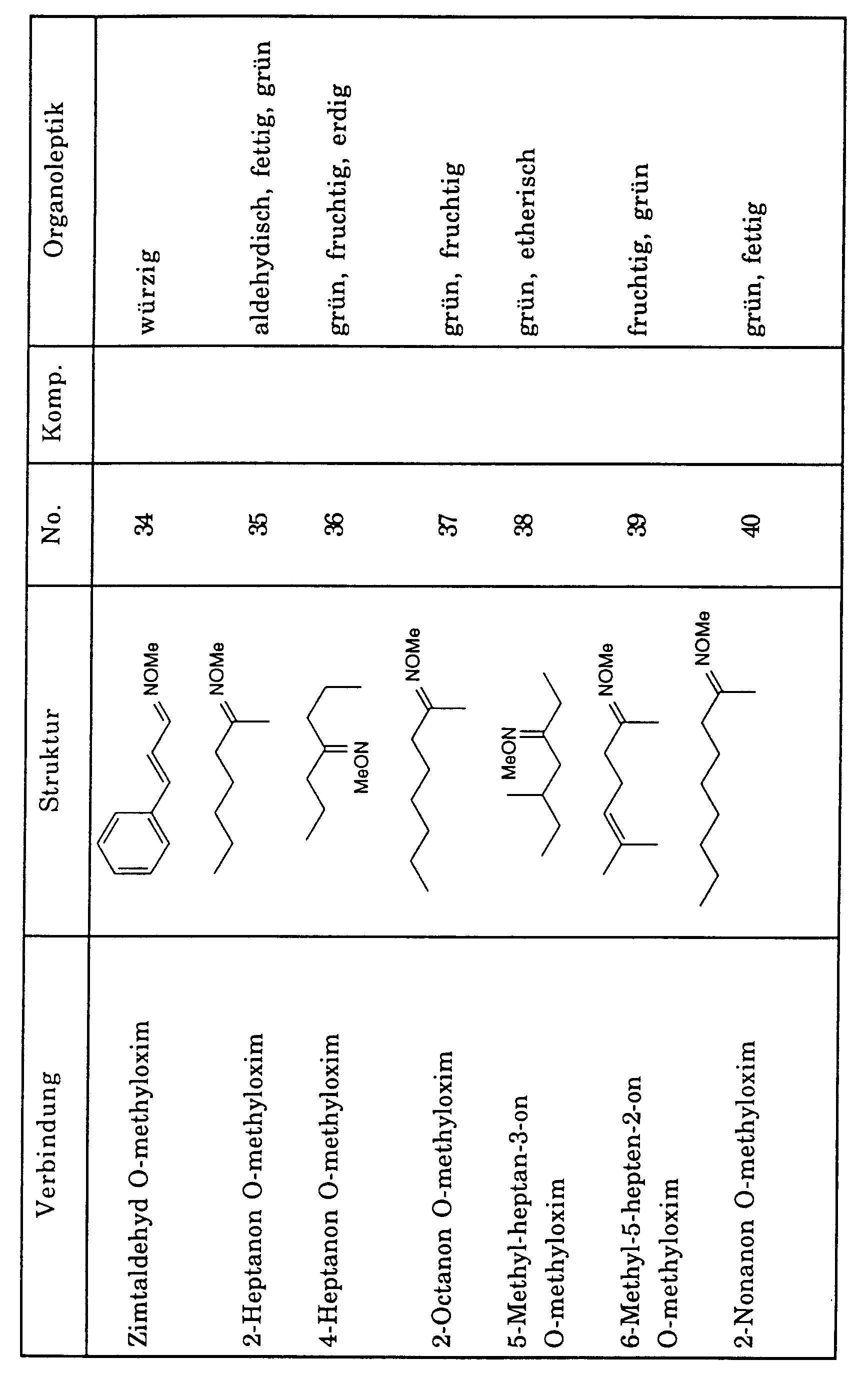

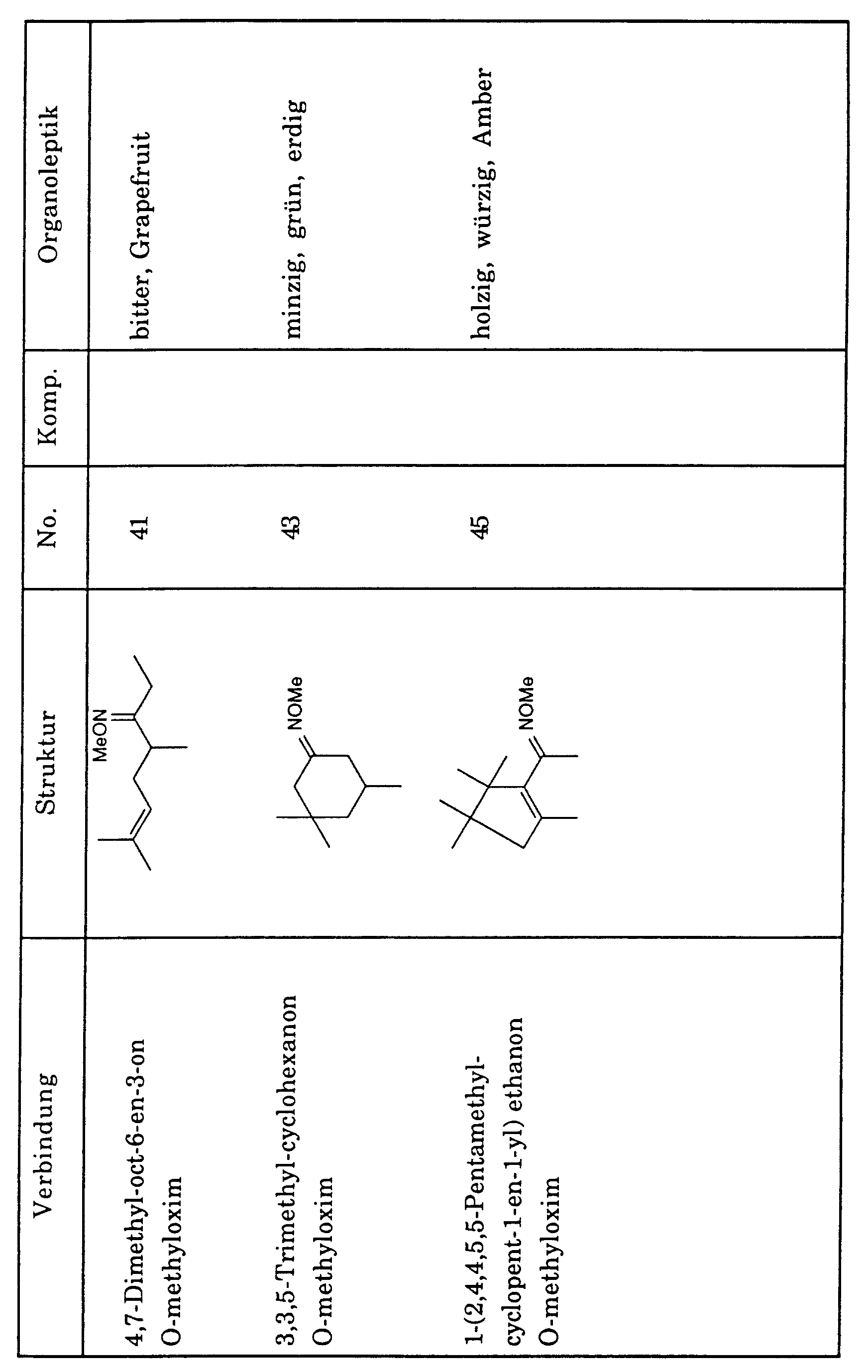

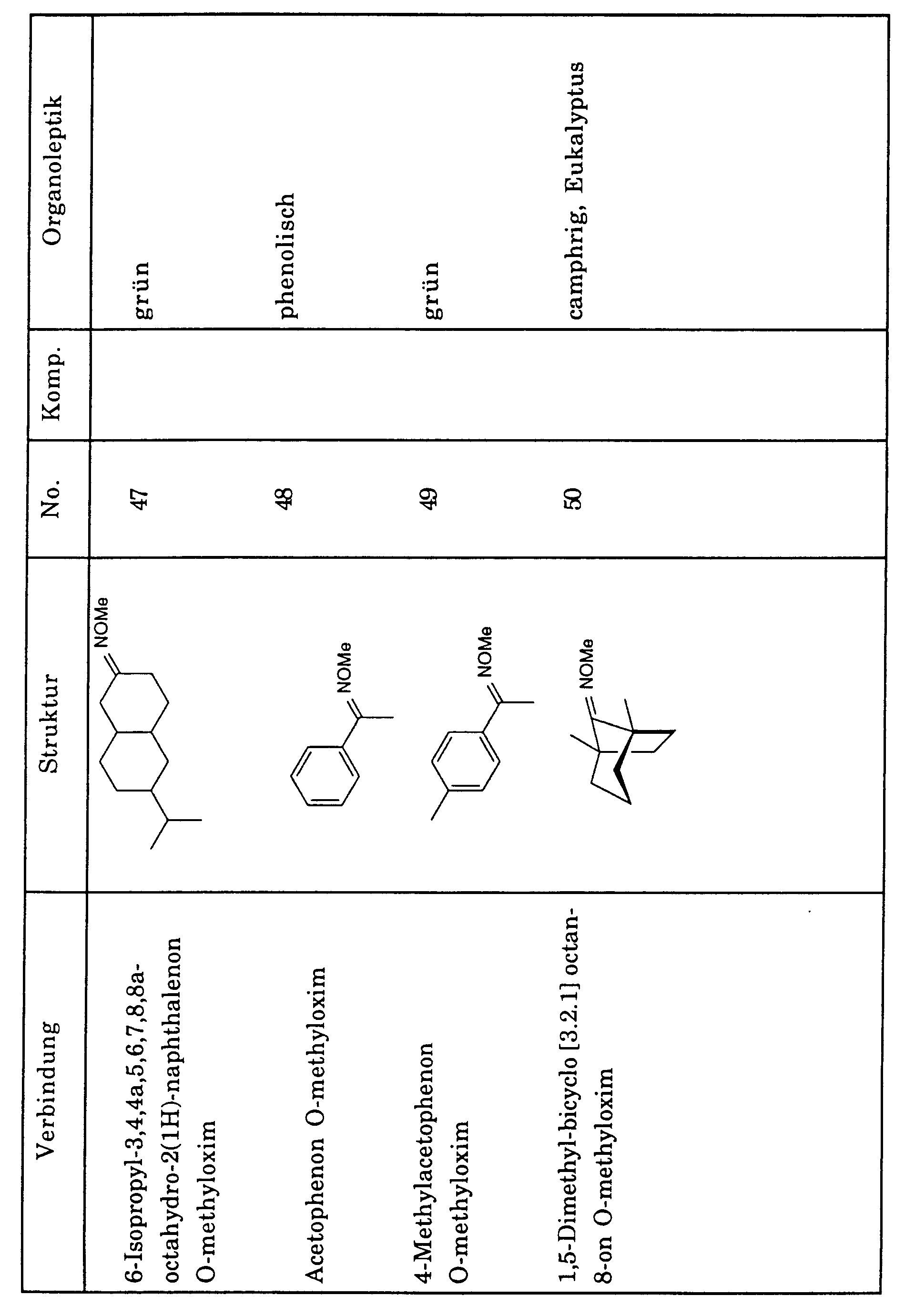

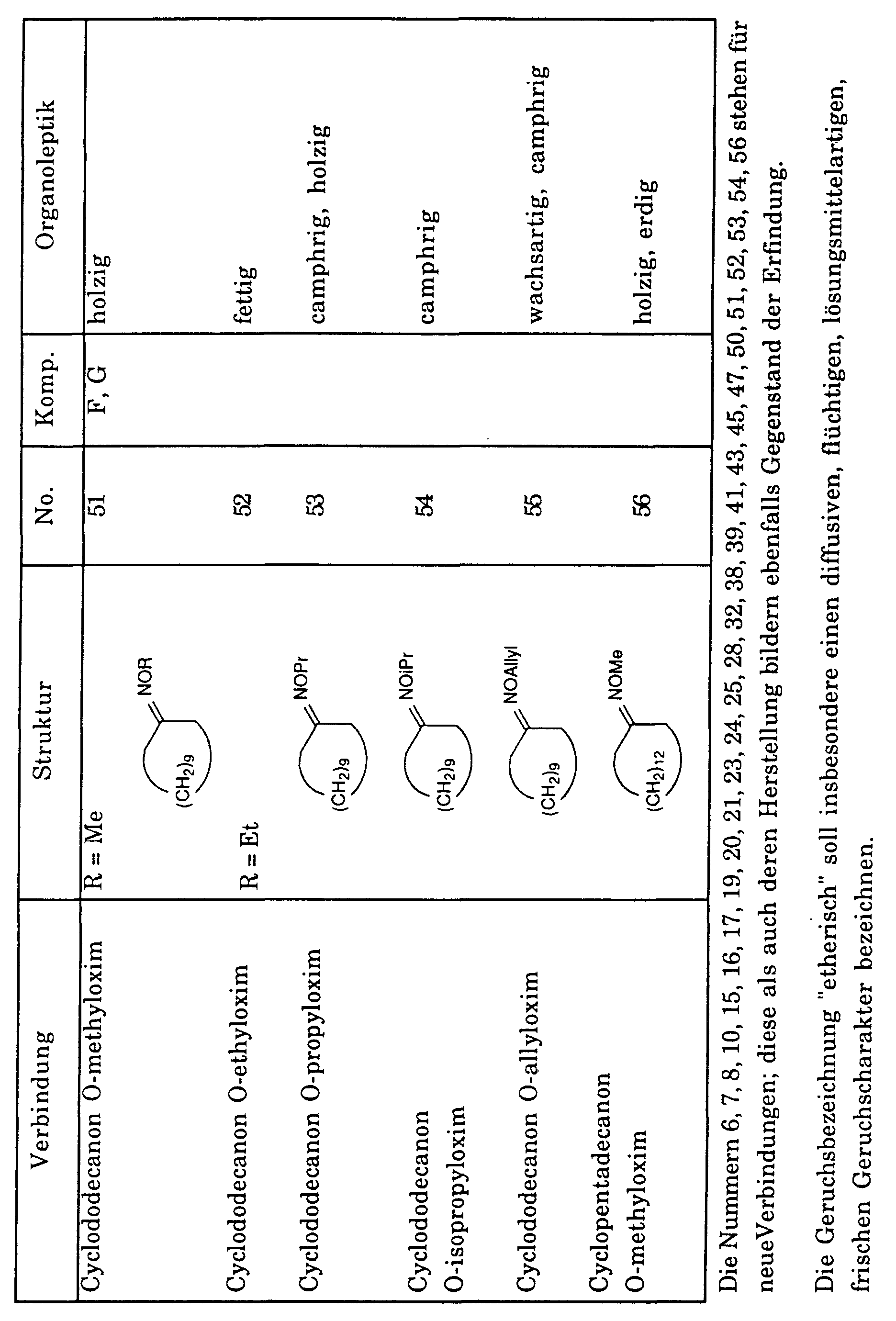

Im Vordergrund des Interesses stehen - in der angegebenen Reihenfolge:

| Verbindung | Riechstoffkompositionen | Geschmackstoffkompositionen |
|---|---|---|
| 3-Methylbutanal O-methyloxim (4) | | X |
| 2-Methylbutanal O-methyloxim (5) | | X |
| 2,4-Dimethyl-cyclohex-3-en-1-carbaldehyd O-methyloxim (19) | X | |
| p-Toluylaldehyd O-methyloxim (27) | X | X |
| 4-Heptanon O-methyloxim (36) | X | |
| 3-Aethoxy-4-hydroxy-benzaldehyd-O-methyloxim (32) | X | |
| 4-Methoxybenzaldehyd O-methyloxim (30) | X | |
| (E)-2-Hexenal O-methyloxim (9) | X | |
| Cyclododecanon O-methyloxim (51) | X | |

Die Bevorzugung dieser einzelnen Verbindungen ergibt sich aufgrund folgender Eigenschaften:
(4) die natürlich Grünnote, der tiefe Schwellenwert
(5) die natürliche Note, der tiefe Schwellenwert
(19) die natürliche Grünnote
(27) die fruchtige frische Note
(36) die grüne fruchtige Note
(32) ein stabiler Vanillinersatz
(30) ein stabilisiertes Anisaldehyd
(9) fruchtige und grüne Kopfnote
(51) erbringt Eleganz in Holznoten
über Oximäther ist insbesondere folgendes bekannt:
I. R.Kaiser, in "Perfumes", P.M.Müller u. D. Lamparsky, 213, Elsevier (1991) 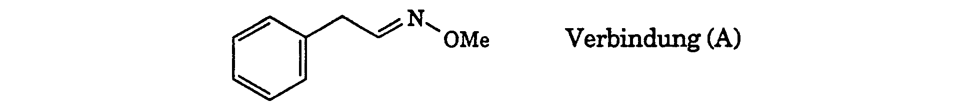
   Geruch nach Phenylacetaldehyd,
   Phenylpropionaldehyd,
   Phenylalkyl methyl aether.
II. P. Johnson, in "Zeitschrift für die Fett-, Oel-, Tensid-, Kosmetik- und Pharmaindustrie", 113 Nr. 6 (1987) 173-175.
   (A) ist rosig.
III. D Joulain and R. Laurent, in "Flavors and Fragrances", (1988) 623-625
   (A) ist in der Blüte von Karo-Karounde vorhanden.
IV. A.L. Morales and C.Duque, in "Aroma Constituents of the Fruit of the Mountain Papaya (Carica pubescens) from Colombia" in J.Agric.Food Chem. 35 (1987) 538-540
   Hier wird die Verbindung (27) mit der vermuteten Struktur 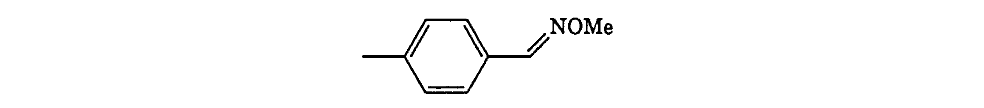 aufgeführt.
V. G.J. Karabatsos and N.Hsi, in Tetrahedron, 23, (1967) 1079-1095 "Confirmations and configurations of oxime O-methyl ethers" führen eine Reihe von Oximäthern auf, die "sweet smelling liquids" sind.

I, II und III handeln im Gegensatz zu den Verbindungen der allgemeinen Formel I von blumigen Noten.

Gemäss IV besitzen die für das Aroma der "Mountain Papaya" verantwortlichen Körper keine stickstoffhaltigen Strukturen, sondern sind Aethylbutyrat, Butanol, Aethylacetat, Methylbutyrat und Butylacetat.

V beinhaltet eine rein strukturelle Studie ohne irgenswelche Verwendungsangaben oder auch nur einen Hinweis auf eine solche Verwendung der hier erwähnten Oximäther.

### Beispiel 1

### Herstellung von 2,4-Dimethyl-cyclohex-3-en-1-carbaldehyd O-methyloxim (19)

Eine Lösung von 133 g (1,59 Mole) O-Methylhydroxylamin und 216 g (1,59 Mole) Natriumacetat triacetat (NaOAc•3H₂O) in 1 l Wasser wird unter Rühren während 30 Minuten mit 200 g (1,45 Mole) Cyclal C® (2,4-Dimethyl-3-cyclohexen-carbaldehyd) versetzt. Das Gemisch wird während 15 Stunden bei Raumtemperatur gut gerührt und anschliessend mit 2 l Ether extrahiert. Die organische Phase wird 2 mal mit Eiswasser (je 1 l), als dann mit halbgesättigter, wässriger NaHCO₃-Lösung (1 l) gewaschen.

Der organische Extrakt wird mit MgSO₄ getrocknet und nach der Filtration am Vakuum aufkonzentriert. Destillation über eine 10-cm-Widmerkolonne bei 50 mbar ergibt 210,2 g (87%) farbloses Produkt (Sdp. 112°C bei 50 mbar).
MS: 167 (M^{+•}, 9)
IR: 1055 cm⁻¹ (stark)
¹H-NMR (200 MH₃, CDCl₃): 3.83 (s, OCH₃), 6.50 (d, J = 8 H_{z}, H-C=N Z-Isomer), 7.27 (d, J = 8 H_{z}, H-C=N E-Isomer).
Geruch: grün, fruchtig

Auf analoge Weise werden erhalten:

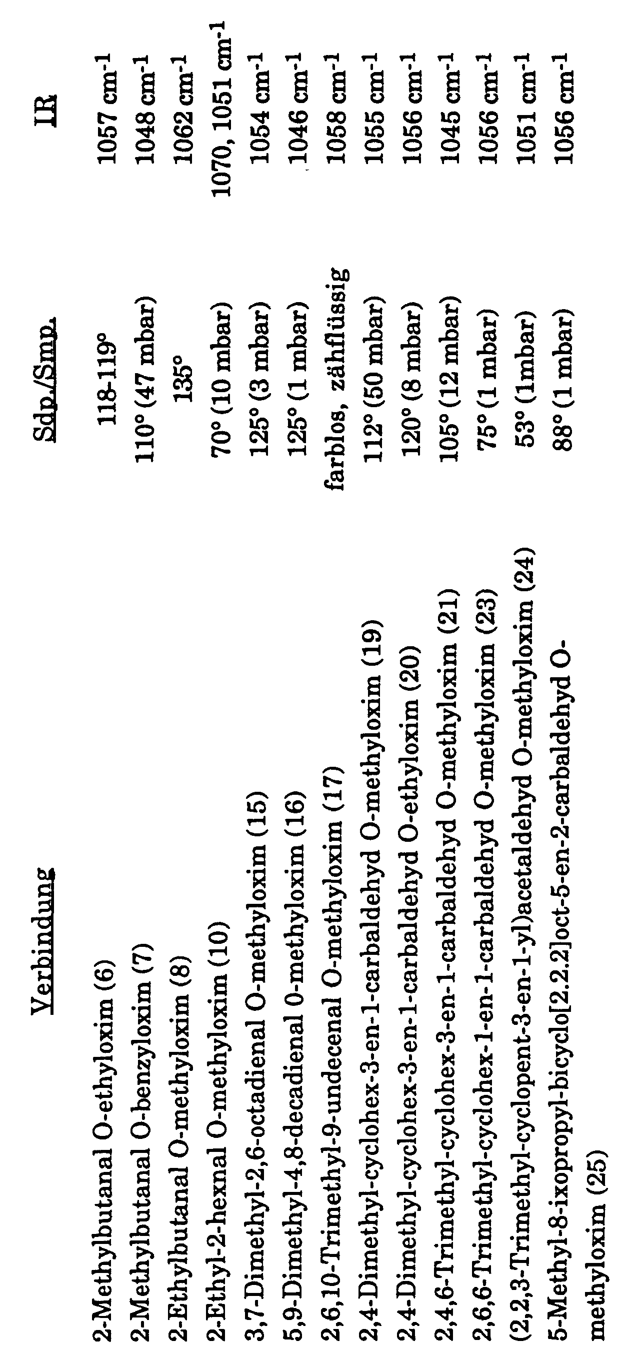

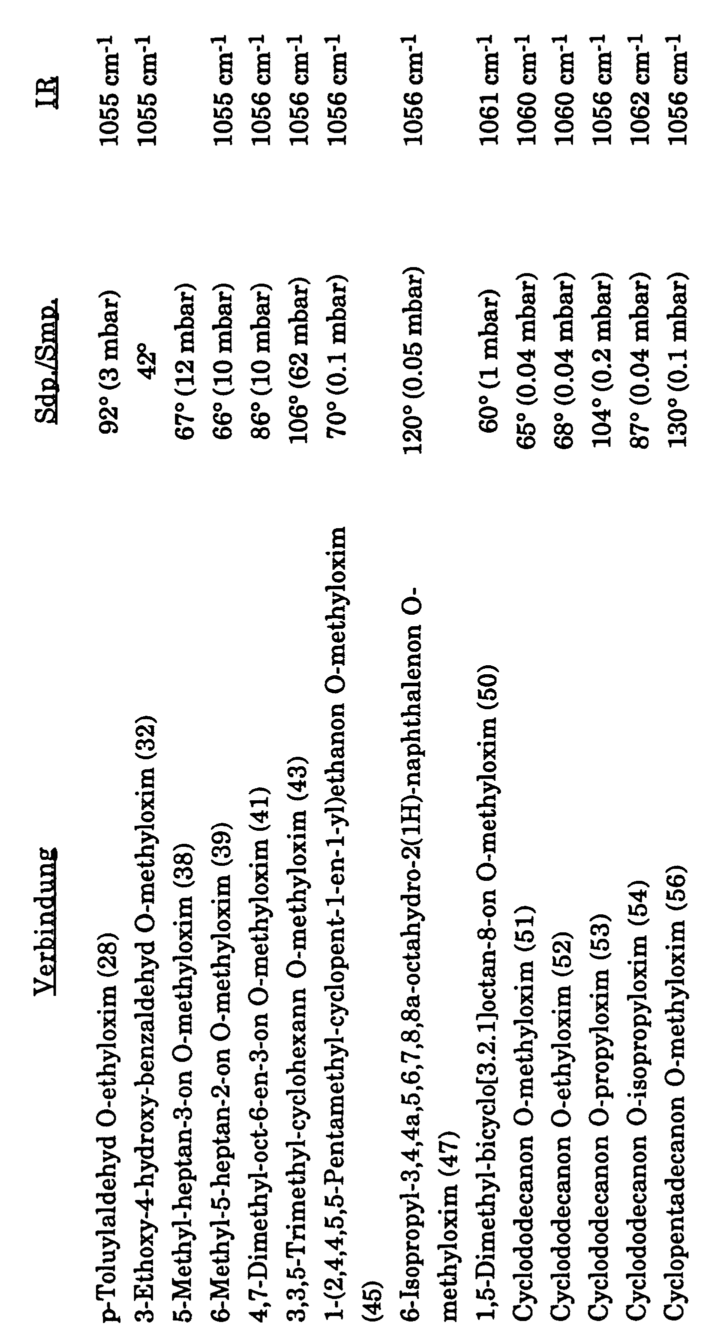

### Beispiel 2

### Herstellung von Cyclododecanon O-methyloxim (51)

Eine Suspension von 1,73 g Natriumhydrid (36 mMol, 50% Dispersion in Mineralöl) in 100 ml DME wird bei 70°C langsam mit einer Lösung von 5.91 g (30 mMol) Cyclododecanonoxim im 100 ml DME versetzt, dann 80 Minuten bei derselben Temperatur gerührt, worauf 5,1 g (36 mMol) Methyliodid bei 40°C zugetropft werden.

Das Gemisch wird 1 Stunde bei 40°C gerührt, mit 300 ml Ether verdünnt und fünfmal mit 250 ml Eiswasser gewaschen.

Die organische Phase wird getrocknet (MgSO₄), filtriert und eingedampft. Der Rückstand wird in Hexan aufgenommen, durch Kieselgel filtriert und anschliessend destilliert.

Man erhält 1,90 g (30%) einer farblosen Flüssigkeit von holzigem Geruch.

### Beispiel 3

### Kompositionen

In den Kompositionen A bis I beziehen sich die in Klammern gesetzten Nummern auf die Tabelle I.

### A. Klassisches Eau de Cologne

| | Gewichtsteile |
|---|---|
| Verbindung (19) | 15.00 |
| Nerylacetat | 5.00 |
| Terpenylacetat | 35.00 |
| Bergamotteessenz | 400.00 |
| Citronenessenz | 120.00 |
| Citronellol extra | 10.00 |
| Dipropylenglykol | 440.00 |
| Estragol | 2.00 |
| Eucalyptus Globulus essenz chines. | 10.00 |
| Eugenol | 23.00 |
| Galaxolide 50 % in Benzylbenzoat | 10.00 |
| Geraniol | 20.00 |
| Geraniumessenz | 10.00 |
| Hydroxycitronellal | 10.00 |
| Lavandinöl | 100.00 |
| Lemarome N (Neral/Citralgemisch) | 30.00 |
| Mandarinenöl | 30.00 |
| Nerol | 10.00 |
| Neroliessenz | 30.00 |
| Orangenessenz | 200.00 |
| Oranger fleurs (2-Acetylnaphthalin) | 15.00 |
| Terpineol | 20.00 |
| Thibetolide (Ω-Pentadecalacton) | 5.00 |
| | Total 1550.00 |

In diesem klassischen leicht würzigen Eau de Cologne erbringt (19) die frischen grüne Blumennote.

### B. Kosmetik-Parfum

| | Gewichtsteile |
|---|---|
| Verbindung (19) | 20.00 |
| Benzylacetat | 40.00 |
| Dimethylbenzylacetat | 40.00 |
| Geranylacetat | 30.00 |
| Phenylethylalkohol | 100.00 |
| Bergamotteessenz | 100.00 |
| α-Isomethylionon | 80.00 |
| Cyclohexal | 40.00 |
| Dipropylenglykol | 80.00 |
| Eugenol | 20.00 |
| Galaxolide 50 % (Diethylphthalat) | 40.00 |
| Gardenol (Methylphenylcarbinylacetat) | 10.00 |
| Geraniol | 50.00 |
| Hedione (Methyldihydrojasmonat) | 40.00 |
| Heliotropin krist. | 20.00 |
| Hydroxycitronellal | 50.00 |
| Methyl-cedryl-keton | 60.00 |
| Methyl-isoeugenol | 10.00 |
| Musk Ketone (4-tert.Butyl-3,5-dinitro-2,6- | 50.00 |
| dimethyl-acetophenon | |
| Benzylsalicylat | 100.00 |
| Thibetolide | 20.00 |
| | Total 1000.00 |

In dieser würzigen Blumennote erbringt (19) den natürlichen Frischecharakter sowie eine Verstärkung des blumigen Charakters.

### C. Eau de Cologne

| | Gewichtsteile |
|---|---|
| Verbindung (27) | 20.0000 |
| Benzylacetat | 60.0000 |
| Acetylisoeugenol | 10.0000 |
| α-Hexylzimtaldehyd | 80.0000 |
| Bergamottebase | 250.0000 |
| Ethylenbrassylat | 80.0000 |
| Citronenessenz Ital. Extra | 80.0000 |
| Dipropylenglykol | 10.0000 |
| Fixolide | 40.0000 |
| Isoraldeine (Isomethyl-α-ionon) | 50.0000 |
| Jasminbase | 80.0000 |
| Methyl-cedryl-keton | 50.0000 |
| Nerolibase | 100.0000 |
| Orangen-essenz Florida | 50.0000 |
| Petitgrain-essenz Paraguay | 20.0000 |
| Sandalore | 20.0000 |
| | Total 1000.0000 |

In diesem klassischen Eau de Cologne erbringt erst (27) den blumigen Akkord und die vollendete Frische.

### D. Weichmacher-Parfum

| | Gewichtsteile |
|---|---|
| Verbindung (27) | 10.0000 |
| Benzylacetat | 40.0000 |
| Geranylacetat | 40.0000 |
| cis-3-Hexenylacetat | 2.0000 |
| p-tert.-Butyl cyclohexylacetat | 60.0000 |
| Zimtalkohol | 30.0000 |
| Phenylethyl-alkohol | 80.0000 |
| Hexyl-zimtaldehyd | 120.0000 |
| Phenylacetaldehyd 85% in Phenylethylalkohol | 1.0000 |
| Bergamotte base | 100.0000 |
| Zedernholzöl virgin. | 30.0000 |
| Dimethylbenzylcarbinylbutyrat | 5.0000 |
| Citronellol extra | 40.0000 |
| Cumarin crist. | 15.0000 |
| δ-Decalacton | 4.0000 |
| Dipropylenglykol | 26.8000 |
| Ethyl-vanillin | 0.2000 |
| Eugenol | 15.0000 |
| Evernyl | 3.0000 |
| Fixolide | 50.0000 |
| Galbanum-essenz | 1.0000 |
| Hydroxycitronellal synt. | 30.0000 |
| Indolene | 2.0000 |
| Lilial | 40.0000 |
| Mandarinen essenz | 10.0000 |
| Methyl-cedryl-keton | 50.0000 |
| Methyl diantilis | 10.0000 |
| Nonadyl | 20.0000 |
| Amyl-salicylat | 50.0000 |
| Benzyl-salicylat | 80.0000 |
| α-Terpineol | 30.0000 |
| Undecavertol (4-Methyl-dec-3-en-5-ol) | 5.0000 |
| | Total 1000.0000 |

(27) vervollständigt hier das Blumenbouquet mit dem gewünschten Frischeffekt.

### E Kosmetik-Parfum

| | Gewichtsteile |
|---|---|
| Verbindung (27) | 15.0000 |
| Benzylacetat | 80.0000 |
| Geranylacetat | 50.0000 |
| Phenylethyl alkohol | 120.0000 |
| Hexylzimtaldehyd | 200.0000 |
| Undecylenaldehyd | 0.3000 |
| Phenylacetaldehyd 85% in Phenylethylalkohol | 2.0000 |
| Zedernholzöl Texas rect. | 50.0000 |
| Dipropylenglykol | 37.7000 |
| Evernyl | 5.0000 |
| Fixolide | 50.0000 |
| Geraniol | 60.0000 |
| Nelkenknospen essenz | 10.0000 |
| Heliotropin | 10.0000 |
| Hydroxycitronellal | 120.0000 |
| Isoraldeine 95% | 30.0000 |
| Jasmonyl LG (Octylcrotonylacetat) | 40.0000 |
| Mandarinen essenz | 20.0000 |
| Benzyl salicylat | 70.0000 |
| Hexenyl-3-cis salicylat | 15.0000 |
| Cetonial [3-(3,4-Methylendioxyphenyl)-2- | 15.0000 |
| methylpropanal] | |
| | Total 1000.0000 |

Die hier vorherrschende blumige Tonalität und Frische ist bedingt durch die Anwesenheit von (27)

### F. Eau de Toilette feminine

| | Gewichtsteile |
|---|---|
| Verbindung (51) | 60.0000 |
| Benzylacetat | 80.0000 |
| Phenylethyl alkohol | 120.0000 |
| α-Hexylzimtaldehyd | 160.0000 |
| Undecylenaldehyd | 0.3000 |
| Phenylacetaldehyd 85% in Phenylethylalkohol | 2.0000 |
| Bergamotte-base | 100.0000 |
| Dipropylenglykol | 42.7000 |
| Evernyl | 2.0000 |
| Geraniol | 60.0000 |
| Nelkenknospenöl | 8.0000 |
| Hedione | 80.0000 |
| Heliotropine | 5.0000 |
| Hydroxycitronellal | 120.0000 |
| Isoraldeine 95% | 20.0000 |
| Mandarinen essenz | 15.0000 |
| Benzyl-salicylat | 80.0000 |
| cis-3-Hexenyl-salicylat | 10.0000 |
| Thibetolide | 20.0000 |
| Cetonial | 15.0000 |
| | Total 1000.0000 |

Die obige Komposition gewinnt durch Anwesenheit von (51) beträchtlich an Eleganz & Volumen.

### G. Komposition für Shampoos

| | Gewichtsteile |
|---|---|
| Verbindung (51) | 70.0000 |
| Benzylacetat | 60.0000 |
| Phenylethyl-alkohol | 150.0000 |
| α-Hexylzimtaldehyd | 100.0000 |
| Undecylenaldehyd | 5.0000 |
| Methyl-anthranilat | 5.0000 |
| Bergamotte-base | 200.0000 |
| Dipropylenglykol | 80.0000 |
| Eugenol | 20.0000 |
| Fixolide | 50.0000 |
| Gardenol | 5.0000 |
| Isoeugenol | 10.0000 |
| Isoraldeine | 20.0000 |
| Linalool synt. | 50.0000 |
| Methyl cedryl keton | 50.0000 |
| Pêche pure (γ-Undecalacton) | 20.0000 |
| Benzyl salicylat | 100.0000 |
| cis-3-Hexenyl-salicylat | 20.0000 |
| Vanillin | 3.0000 |
| | Total 1000.0000 |

Die Zusatz von (51) zum obigen blumigen Akkord bewirkt eine feine holzig-ambrige Tonalität der Komposition.

### H. Ein Kiwi-Aroma kann folgende Zusammensetzung aufweisen:

| | Gewichtsteile |
|---|---|
| Triacetin (Glycerintriacetat) | 878.00 |
| Ethyl acetoacetat | 10.00 |
| Ethyl butyrat | 50.00 |
| Ethyl acetat | 20.00 |
| Amyl-iso-acetat | 3.00 |
| Acetaldehyd | 5.00 |
| E-2-Hexenal | 3.00 |
| Hexyl-alkohol | 5.00 |
| E-2-Hexenol | 2.00 |
| z-3-Hexenol / Blätteralkohol | 20.00 |
| Verbindung (5) | 4.00 |
| Total | 1000.000000 |

Das Aroma besticht durch fruchtige natürliche Noten mit herben Grünnoten.

### I. Aroma für Mundpflegemittel

| | Gewichtsteile |
|---|---|
| L-Menthol | 200 |
| Krauseminzöl | 100 |
| Pfefferminzöl chin. rect. | 200 |
| Pfefferminzöl Italo-Mitcham | 340 |
| Anethol synth. | 130 |
| Eucalyptol | 30 |
| Verbindung (5) | 10 |
| Total | 1000 |

Das Aroma besticht durch seine grüne und natürliche Note.

## Patentansprüche

1. Riech- und/oder Geschmackstoffkomposition, **gekennzeichnet durch** einen Gehalt an einer Verbindung der Formel 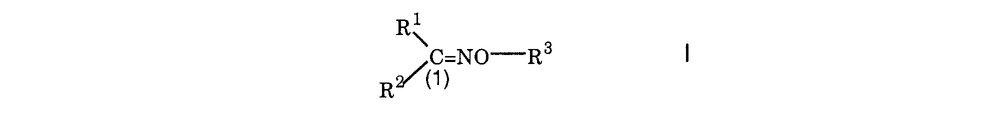
worin R¹, R² H, Alkyl, Alkenyl,
gegebenenfalls substituiertes Cycloalkyl,
gegebenenfalls substituiertes Cycloalkyl-alkyl,
gegebenenfalls substituiertes Cycloalkyl-alkenyl,
gegebenenfalls substituiertes Cycloalkenyl,
gegebenenfalls substituiertes Cycloalkenyl-alkyl oder
gegebenenfalls substituiertes Phenyl, substituiertes Benzyl,
substuiertes Phenethyl, Phenylalkenyl,
ein bicyclischer Rest,
oder R¹+R²+ Kohlenstoffatom C₁ zusammen gebenenfalls substituiertes Cycloalkyl oder ein bicyclischer Rest und R³ Alkyl mit 1-4 C Atomen oder Alkenyl mit 2-4 C Atomen oder Benzyl bedeuten.

2. Riech- und/oder Geschmackstoffkomposition gemäss Anspruch 1, enthaltend eine Verbindung der Formel I,
worin R¹ + R² + Kohlenstoffatom C₁ gegebenenfalls subst. Cycloalkyl, und
R³ Alkyl mit 1-4 C Atomen bedeuten.

3. Riech- und/oder Geschmackstoffkomposition gemäss Anspruch 1, enthaltend eine Verbindung ausgewählt aus der Gruppe
6-Methyl-5-hepten-2-on O-methyloxim,
6-Isopropyl-3,4,4a,5,6,7,8,8a-octahydro-2(14)-naphthalenon O-methyloxim,
1-(2,4,4,5,5-Penta-methyl-cyclopent-1-en-1-yl)ethanon O-methyloxim,
5-Methyl-heptan-3-on O-methyloxim,
2,4-Dimethyl-cyclohex-3-en-1-carbaldehyd O-methyloxim,
3,7-Dimethyl-2,6-octadienal O-methyloxim,
2,4,6-Trimethyl-cyclohex-3-en-1-carbaldehyd O-methyloxim,
2,6,6-Trimethyl-cyclohex-1-en-1-carbaldehyd O-methyloxim,
2-Ethyl-hex-2-enal O-methyloxim,
(2,2,3-Trimethyl-cyclo-pent-3-en-1-yl)acetaldehyd O-methyloxim und
5-Methyl-8-isopropyl-bicyclo [2.2.2.] oct-5-en-2-carbaldehyd O-methyloxim.

4. Riech- und/oder Geschmackstoffkomposition gemäss Anspruch 1, enthaltend eine Verbindung ausgewählt aus der Gruppe
3-Methylbutanal O-methyloxim,
2-Methylbutanal O-methyloxim,
2,4-Dimethyl-cyclohex-3-en-1-carbaldehyd O-methyloxim,
p-Toluylaldehyd O-methyloxim,
4-Heptanon O-methyloxim,
3-Aethoxy-4-hydroxy-benzaldehyd-O-methyloxim,
4-Methoxybenzaldehyd O-methyloxim,
E-2 Hexenal O-methyloxim und
Cyclododecanon O-methyloxim.

5. Riech- und/oder Geschmackstoffkomposition, enthaltend eine Verbindung ausgewählt aus der Gruppe
Propanal O-methyloxim,
Butanal O-methyloxim,
3-Methylbutanal O-methyloxim,
2-Methylbutanal O-methyloxim und
Cyclohexancarbaldehyd O-metyloxim.

6. Verwendung der Verbindungen der Formel 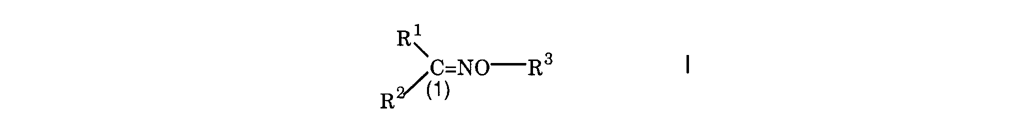
worin R¹, R² H, Alkyl, Alkenyl,
gegebenenfalls substituiertes Cycloalkyl,
gegebenenfalls substituiertes Cycloalkyl-alkyl,
gegebenenfalls substituiertes Cycloalkyl-alkenyl,
gegebenenfalls substituiertes Cycloalkenyl,
gegebenenfalls substituiertes Cycloalkenyl-alkyl, oder
gegebenenfalls substituiertes Phenyl, substituiertes Benzyl,
substuiertes Phenethyl, Phenylalkenyl,
ein bicyclischer Rest,
oder R¹+R²+ Kohlenstoffatom C₁ zusammen gebenenfalls substituiertes Cycloalkyl oder ein bicyclischer Rest und
R³ Alkyl mit 1-4 C Atomen oder Alkenyl mit 2-4 C Atomen oder Benzyl bedeuten,
als Riech- und/oder Geschmackstoffe.

7. Verwendung der Verbindungen der Formel I
worin R¹ + R² + Kohlenstoffatom C₁ gegebenenfalls subst. Cycloalkyl, und
R³ Alkyl mit 1-4 C Atomen bedeuten,
als Riech- und/oder Geschmackstoffe.

8. Verwendung von
6-Methyl-5-hepten-2-on O-methyloxim,
6-Isopropyl-3,4,4a,5,6,7,8,8a-octahydro-2(14)-naphthalenon O-methyloxim,
1-(2,4,4,5,5-Penta-methyl-cyclopent-1-en-1-yl)ethanon O-methyloxim,
5-Methyl-heptan-3-on O-methyloxim,
2,4-Dimethyl-cyclohex-3-en-1-carbaldehyd O-methyloxim,
3,7-Dimethyl-2,6-octadienal O-methyloxim,
2,4,6-Trimethyl-cyclohex-3-en-1-carbaldehyd O-methyloxim,
2,6,6-Trimethyl-cyclohex-1-en-1-carbaldehyd O-methyloxim,
2-Ethyl-hex-2-enal O-methyloxim,
(2,2,3-Trimethyl-cyclo-pent-3-en-1-yl)acetaldehyd O-methyloxim oder 5-Methyl-8-isopropyl-bicyclo [2.2.2.]oct-5-en-2-carbaldehyd O-methyloxim
als Riech- und/oder Geschmackstoff.

9. Verwendung von
3-Methylbutanal O-methyloxim,
2-Methylbutanal O-methyloxim,
2,4-Dimethyl-cyclohex-3-en-1-carbaldehyd O-methyloxim,
p-Toluylaldehyd O-methyloxim,
4-Heptanon O-methyloxim,
3-Aethoxy-4-hydroxy-benzaldehyd-O-methyloxim,
4-Methoxybenzaldehyd O-methyloxim,
E-2 Hexenal O-methyloxim oder
Cyclododecanon O-methyloxim
als Riech- und/oder Geschmackstoff.

10. Verwendung von
Propanal O-methyloxim,
Butanal O-methyloxim,
3-Methylbutanal O-methyloxim,
2-Methylbutanal O-methyloxim oder
Cyclohexancarbaldehyd O-metyloxim
als Riech- und/oder Geschmackstoff.

11. Eine Verbindung ausgewählt aus der Gruppe
6-Methyl-5-hepten-2-on O-methyloxim,
6-Isopropyl-3,4,4a,5,6,7,8,8a-octahydro-2(14)-naphthalenon O-methyloxim,
1-(2,4,4,5,5-Penta-methyl-cyclopent-1-en-1-yl)ethanon O-methyloxim,
5-Methyl-heptan-3-on O-methyloxim,
2,4-Dimethyl-cyclohex-3-en-1-carbaldehyd O-methyloxim,
4-(4-Methyl-3-penten-1-yl) cyclohex-3-en-1-carbaldehyd O-methyloxim,
3,7-Dimethyl-2,6-octadienal O-methyloxim,
2,4,6-Trimethyl-cyclohex-3-en-1-carbaldehyd O-methyloxim,
2,6,6-Trimethyl-cyclohex-1-en-1-carbaldehyd O-methyloxim,
2-Ethyl-hex-2-enal O-methyloxim,
(2,2,3-Trimethyl-cyclo-pent-3-en-1-yl)acetaldehyd O-methyloxim und
5-Methyl-8-isopropyl-bicyclo [2.2.2.]oct-5-en-2-carbaldehyd O-methyloxim.

12. Eine Verbindung, ausgewählt aus der Gruppe
2-Methylbutanal O-ethyloxim,
2-Methylbutanal O-benzyloxim,
2-Ethylbutanal O-methyloxim,
5,9-Dimethyl-4,8-decadienal O-methyloxim,
2,6,10-Trimethyl-9-undecenal O-methyloxim,
2,4-Dimethyl-cyclohex-3-en-1-carbaldehyd O-ethyloxim,
p-Toluylaldehyd-O-ethyloxim,
3-Ethoxy-4-hydroxy-benzaldehyd-O-methyloxim,
5-Methyl-heptan-3-on O-methyloxim,
4,7-Dimethyl-oct-6-en-3-on O-methyloxim,
3,3,5-Trimethyl-cyclohexanon O-methyloxim,
1,5-Dimethyl-bicyclo[3.2.1]octan-8-on O-methyloxim,
Cyclododecanon O-methyloxim,
Cyclododecanon O-ethyloxim,
Cyclododecanon O-propyloxim,
Cyclododecanon O-isopropyloxim und
Cyclopentadecanon O-methyloxim.

## Claims

1. Odoriferous substance and/or flavouring agent composition, **characterised by** containing a compound of the formula 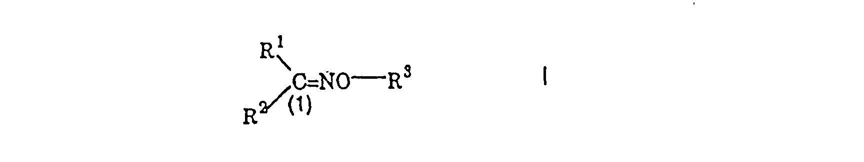 wherein R¹, R² denote H, alkyl, alkenyl, optionally substituted cycloalkyl, optionally substituted cycloalkyl-alkyl, optionally substituted cycloalkyl-alkenyl, optionally substituted cycloalkenyl, optionally substituted cycloalkenyl-alkyl or optionally substituted phenyl, substituted benzyl, substituted phenethyl, phenylalkenyl, a bicyclic radical, or R¹+R²+carbon atom C₁ together denote optionally substituted cycloalkyl or a bicyclic radical, and R³ denotes alkyl with 1-4 C atoms or alkenyl with 2-4 C atoms, or benzyl.

2. Odoriferous substance and/or flavouring agent composition according to claim 1, containing a compound of the formula I,
wherein R¹+R²+carbon atom C; denote optionally substituted cycloalkyl, and
R³ denotes alkyl with 1-4 C atoms.

3. Odoriferous substance and/or flavouring agent composition according to claim 1, containing a compound selected from the group:
6-methyl-5-hepten-2-one O-methyloxime,
6-isopropyl-3,4,4a,5,6,7,8,8a-octahydro-2(1H)naphthalenone O-methyloxime,
1-(2,4,4,5,5-pentamethylcyclopent-1-en-1-yl)ethanone O-methyloxime,
5-methylheptan-3-one O-methyloxime,
2,4-dimethylcyclohex-3-ene-1-carbaldehyde O-methyloxime,
3,7-dimethyl-2,6-octadienal O-methyloxime,
2,4,6-trimethylcyclohex-3-ene-1-carbaldehyde O-methyloxime,
2,6,6-trimethylcyclohex-1-ene-1-carbaldehyde O-methyloxime,
2-ethylhex-2-enal O-methyloxime,
(2,2,3-trimethylcyclopent-3-en-1-yl)acetaldehyde O-methyloxime, and
5-methyl-8-isopropylbicyclo[2.2.2]oct-5-ene-2-carbaldehyde O-methyloxime.

4. Odoriferous substance and/or flavouring agent composition according to claim 1, containing a compound selected from the group:
3-methylbutanal O-methyloxime,
2-methylbutanal O-methyloxime,
2,4-dimethylcyclohex-3-ene-1-carbaldehyde O-methyloxime,
p-toluylaldehyde O-methyloxime,
4-heptanone O-methyloxime,
3-ethoxy-4-hydroxybenzaldehyde O-methyloxime
4-methoxybenzaldehyde O-methyloxime,
E-2-hexenal O-methyloxime, and
cyclododecarione O-methyloxime.

5. Odoriferous substance and/or flavouring agent composition containing a compound selected from the group:
propanal O-methyloxime,
butanal O-methyloxime,
3-methylbutanal O-methyloxime,
2-methylbutanal O-methyloxime, and
cyclohexanecarbaldehyde O-methyloxime.

6. Use of the compounds of the formula 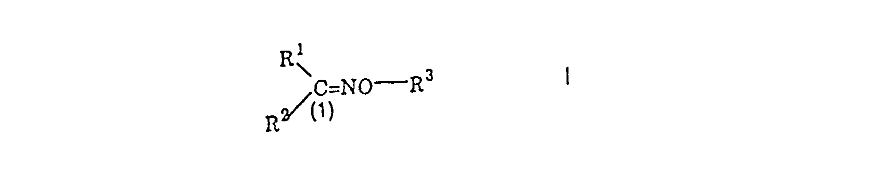 wherein R¹, R² denote H, alkyl, alkenyl, optionally substituted cycloalkyl, optionally substituted cycloalkyl-alkyl, optionally substituted cycloalkyl-alkenyl, optionally substituted cycloalkenyl, optionally substituted cycloalkenyl-alkyl or optionally substituted phenyl, substituted benzyl, substituted phenethyl, phenylalkenyl, a bicyclic radical, or R¹+R²+carbon atom C₁ together denote optionally substituted cycloalkyl or a bicyclic radical, and R³ denotes alkyl with 1-4 C atoms, or alkenyl with 2-4 C atoms, or benzyl,
as odoriferous substances and/or flavouring agents.

7. Use of the compounds of the formula I
wherein R¹+R²+carbon atom C₁ denote optionally substituted cycloalkyl, and
R³ denotes alkyl with 1-4 C atoms,
as odoriferous substances and/or flavouring agents.

8. Use of
6-methyl-5-hepten-2-one O-methyloxime,
6-isopropyl-3,4,4a,5,6,7,8,8a-octahydro-2(1H)naphthalenone O-methyloxime,
1-(2,4,4,5,5-pentamethylcyclopent-1-en-1-yl)ethanone O-methyloxime,
5-methylheptan-3-one O-methyloxime,
2, 4-dimethylcyclohex-3-ene-1-carbaldehyde O-methyloxime,
3,7-dimethyl-2,6-octadienal O-methyloxime,
2,4,6-trimethylcyclohex-3-ene-1-carbaldehyde O-methyloxime,
2,6,6-trimethylcyclohex-1-ene-1-carbaldehyde O-methyloxime,
2-ethylhex-2-enal O-methyloxime,
(2,2,3-trimethylcyclopent-3-en-1-yl)acetaldehyde O-methyloxime, or
5-methyl-8-isopropylbicyclo[2,2,2]oct-5-ene-2-carbaldehyde O-methyloxime
as odoriferous substance and/or flavouring agent.

9. Use of
3-methylbutanal O-methyloxime,
2-methylbutanal O-methyloxime,
2,4-dimethylcyclohex-3-ene-1-carbaldehyde O-methyloxime,
p-toluylaldehyde O-methyloxime,
4-heptanone O-methyloxime,
3-ethoxy-4-hydroxybenzaldehyde O-methyloxime,
4-methoxybenzaldehyde O-methyloxime,
E-2-hexenal O-methyloxime, or
cyclododecanone O-methyloxime
as odoriferous substance and/or flavouring agent.

10. Use of
propanal O-methyloxime,
butanal O-methyloxime,
3-methylbutanal O-methyloxime,
2-methylbutanal O-methyloxime, or
cyclohexanecarbaldehyde O-methyloxime.
as odoriferous substance and/or flavouring agent.

11. A compound selected from the group
6-methyl-5-hepten-2-one O-methyloxime,
6-isopropyl-3,4,4a,5,6,7,8,8a-octahydro-2(1H)naphthalenone O-methyloxime,
1-(2,4,4,5,5-pentamethylcyclopent-1-en-1-yl)ethanone O-methyloxime,
5-methylheptan-3-one O-methyloxime,
2,4-dimethylcyclohex-3-ene-1-carbaldehyde O-methyloxime,
4-(4-methyl-3-penten-1-yl)-cyclohex-3-en-1-carbaldehyd O-methyloxime,
3,7-dimethyl-2,6-octadienal O-methyloxime,
2,4,6-trimethylcyclohex-3-ene-1-carbaldehyde O-methyloxime,
2,6,6-trimethylcyclohex-1-ene-1-carbaldehyde O-methyloxime,
2-ethylhex-2-enal O-methyloxime,
(2,2,3-trimethylcyclopent-3-en-1-yl)acetaldehyde O-methyloxime, and
5-methyl-8-isopropylbicyclo[2.2.2]oct-5-ene-2-carbaldehyde O-methyloxime.

12. A compound selected from the group
2-methylbutanal O-ethyloxime,
2-methylbutanal O-benzyloxime,
2-ethylbutanal O-methyloxime,
5,9-dimethyl-4,8-decadienal O-methyloxime,
2,6,10-trimethyl-9-undecenal O-methyloxime,
2,4-dimethylcyclohex-3-ene-1-carbaldehyde O-ethyloxime,
p-toluylaldehyde O-methyloxime,
3-ethoxy-4-hydroxybenzaldehyde O-methyloxime,
5-methylheptan-3-one O-methyloxime,
4,7-dimethyloct-6-en-3-one O-methyloxime,
3,3,5-trimethylcyclohexanone O-methyloxime,
1,5-dimethylbicyclo[3.2.1]octan-8-one O-methyloxime,
cyclododecanone O-merhyloxine,
cyclododecanone O-ethyloxime,
cyclododecanone O-propyloxime,
cyclododecanone O-isopropyloxime, and
cyclopentadecanone O-methyloxime.

## Revendications

1. Composition d'agents aromatisants et/ou de sapidité, **caractérisée en ce qu'**elle contient un composé de formule 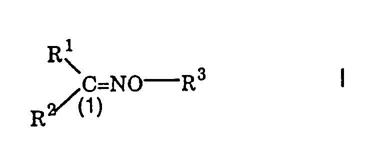 dans laquelle R¹, R² sont chacun H ou un groupe alkyle ou alcényle, cycloalkyle éventuellement substitué, cycloalkylalkyle éventuellement substitué, cycloalkylalcényle éventuellement substitué, cycloalcényle éventuellement substitué, cycloalcénylalkyle éventuellement substitué ou phényle éventuellement substitué, benzyle substitué, phénéthyle substitué, phénylalcényle, un radical bicyclique,
ou encore R¹ + R² + l'atome de carbone C₁ forment ensemble un groupe cycloalkyle éventuellement substitué ou un radical bicyclique, et R³ est un groupe alkyle ayant de 1 à 4 atomes de carbone ou alcényle ayant de 2 à 4 atomes de carbone ou benzyle.

2. Composition d'agents aromatisants et/ou de sapidité selon la revendication 1, contenant un composé de formule I dans laquelle R¹ + R² + l'atome de carbone C₁ représentent un groupe cycloalkyle éventuellement substitué, et R³ est un groupe alkyle ayant de 1 à 4 atomes de carbone.

3. Composition d'agents aromatisants et/ou de sapidité selon la revendication 1, contenant un composé choisi dans l'ensemble suivant :
6-méthyl-5-heptène-2-one-O-méthyloxime,
6-isopropyl-3,4,4a,5,6,7,8,8a-octahydro-2(1H)-naphtalénone-O-méthyloxime,
1-(2,4,4,5,5-pentaméthyl-cyclopent-1-ène-1-yl)éthanone-O-méthyloxime,
5-méthyl-heptane-3-one-O-méthyloxime,
2,4-diméthyl-cyclohex-3-ène-1-carbaldéhyde-O-méthyloxime,
3,7-diméthyl-2,6-octadiénal-O-méthyloxime,
2,4,6-triméthyl-cyclohex-3-ène-1-carbaldéhyde-O-méthyloxime,
2,6,6-triméthyl-cyclohex-1-ène-1-carbaldéhyde-O-méthyloxime,
2-éthyl-hex-2-énal-O-méthyloxime,
(2,2,3-triméthyl-cyclo-pent-3-ène-1-yl)acétaldéhyde-O-méthyloxime et
5-méthyl-8-isopropyl-bicyclo[2.2.2]oct-5-ène-2-carbaldéhyde-O-méthyloxime.

4. Composition d'agents aromatisants et/ou de sapidité selon la revendication 1, contenant un composé choisi dans l'ensemble suivant :
3-méthylbutanal-O-méthyloxime,
2-méthylbutanal-O-méthyloxime,
2,4-diméthyl-cyclohex-3-ène-1-carbaldéhyde-O-méthyloxime,
p-toluylaldéhyde-O-méthyloxime,
4-heptanone-O-méthyloxime,
3-éthoxy-4-hydroxy-benzaldéhyde-O-méthyloxime,
4-méthoxybenzaldéhyde-O-méthyloxime,
E-2-hexénal-O-méthyloxime, et
cyclodécanone-O-méthyloxime.

5. Composition d'agents aromatisants et/ou de sapidité contenant un composé choisi dans l'ensemble suivant :
propanal-O-méthyloxime,
butanal-O-méthyloxime,
3-méthylbutanal-O-méthyloxime,
2-méthylbutanal-O-méthyloxime, et
cyclohexanecarbaldéhyde-O-méthyloxime.

6. Utilisation des composés de formule 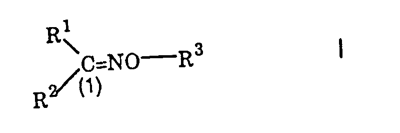 dans laquelle R¹, R² sont chacun H ou un groupe alkyle ou alcényle, cycloalkyle éventuellement substitué, cycloalkylalkyle éventuellement substitué, cycloalkylalcényle éventuellement substitué, cycloalcényle éventuellement substitué, cycloalcénylalkyle éventuellement substitué ou phényle éventuellement substitué, benzyle substitué, phénéthyle substitué, phénylalcényle, un radical bicyclique,
ou encore R¹ + R² + l'atome de carbone C₁ forment ensemble un groupe cycloalkyle éventuellement substitué ou un radical bicyclique, et R³ est un groupe alkyle ayant de 1 à 4 atomes de carbone ou alcényle ayant de 2 à 4 atomes de carbone ou benzyle,
en tant qu'agents aromatisants et/ou de sapidité.

7. Utilisation des composés de formule I, dans laquelle R¹ + R² + l'atome de carbone C₁ représentent un groupe cycloalkyle éventuellement substitué, et R³ est un groupe alkyle ayant de 1 à 4 atomes de carbone, en tant qu'agents aromatisants et/ou de sapidité.

8. Utilisation de
6-méthyl-5-heptène-2-one-O-méthyloxime,
6-isopropyl-3,4,4a,5,6,7,8,8a-octahydro-2(1H)-naphtalénone-O-méthyloxime,
1-(2,4,4,5,5-pentaméthyl-cyclopent-1-ène-1-yl)éthanone-O-méthyloxime,
5-méthyl-heptane-3-one-O-méthyloxime,
2,4-diméthyl-cyclohex-3-ène-1-carbaldéhyde-O-méthyloxime,
3,7-diméthyl-2,6-octadiénal-O-méthyloxime,
2,4,6-triméthyl-cyclohex-3-ène-1-carbaldéhyde-O-méthyloxime,
2,6,6-triméthyl-cyclohex-1-ène-1-carbaldéhyde-O-méthyloxime,
2-éthyl-hex-2-énal-O-méthyloxime,
(2,2,3-triméthyl-cyclo-pent-3-ène-1-yl)acétaldéhyde-O-méthyloxime et
5-méthyl-8-isopropyl-bicyclo[2.2.2]oct-5-ène-2-carbaldéhyde-O-méthyloxime,
en tant qu'agent aromatisant et/ou de sapidité.

9. Utilisation de
3-méthylbutanal-O-méthyloxime,
2-méthylbutanal-O-méthyloxime,
2,4-diméthyl-cyclohex-3-ène-1-carbaldéhyde-O-méthyloxime,
p-toluylaldéhyde-O-méthyloxime,
4-heptanone-O-méthyloxime,
3-éthoxy-4-hydroxy-benzaldéhyde-O-méthyloxime,
4-méthoxybenzaldéhyde-O-méthyloxime,
E-2-hexénal-O-méthyloxime, et
cyclodécanone-O-méthyloxime,
en tant qu'agent aromatisant et/ou de sapidité.

10. Utilisation de
propanal-O-méthyloxime,
butanal-O-méthyloxime,
3-méthylbutanal-O-méthyloxime,
2-méthylbutanal-O-méthyloxime, et
cyclohexanecarbaldéhyde-O-méthyloxime,
en tant qu'agent aromatisant et/ou de sapidité.

11. Composé choisi dans l'ensemble comprenant les composés suivants :
6-méthyl-5-heptène-2-one-O-méthyloxime,
6-isopropyl-3,4,4a,5,6,7,8,8a-octahydro-2(14)-naphtalénone-O-méthyloxime,
1-(2,4,4,5,5-pentaméthyl-cyclopent-1-ène-1-yl)éthanone-O-méthyloxime,
5-méthyl-heptane-3-one-O-méthyloxime,
2,4-diméthyl-cyclohex-3-ène-1-carbaldéhyde-O-méthyloxime,
3,7-diméthyl-2,6-octadiénal-O-méthyloxime,
2,4,6-triméthyl-cyclohex-3-ène-1-carbaldéhyde-O-méthyloxime,
2,6,6-triméthyl-cyclohex-1-ène-1-carbaldéhyde-O-méthyloxime,
2-éthyl-hex-2-énal-O-méthyloxime,
(2,2,3-triméthyl-cyclo-pent-3-ène-1-yl)acétaldéhyde-O-méthyloxime et
5-méthyl-8-isopropyl-bicyclo[2.2.2]oct-5-ène-2-carbaldéhyde-O-méthyloxime.

12. Composé choisi dans l'ensemble comprenant les composés suivants :
2-méthylbutanal-O-éthyloxime,
2-méthylbutanal-O-benzyloxime,
2-éthylbutanal-O-méthyloxime,
5,9-diméthyl-4,8-didécadiénal-O-méthyloxime,
2,6,10-triméthyl-9-undécénal-O-méthyloxime,
2,4-diméthyl-cyclohex-3-ène-1-carbaldéhyde-O-éthyloxime,
p-toluylaldéhyde-O-éthyloxime,
3-éthoxy-4-hydroxy-benzaldéhyde-O-méthyloxime,
5-méthyl-heptane-3-one-O-méthyloxime,
4,7-diméthyl-oct-6-ène-3-one-O-méthyloxime,
3,3,5-triméthyl-cyclohexanone-O-méthyloxime,
1,5-diméthyl-bicyclo[3.2.1]octane-8-one-O-méthyloxime,
cyclododécanone-O-méthyloxime,
cyclododécanone-O-éthyloxime,
cyclododécanone-O-propyloxime,
cyclododécanone-O-isopropyloxime, et
cyclopentadécanone-O-méthyloxime.
